# EUROPEAN PATENT SPECIFICATION

(11) **EP 1 878 436 B1**
(45) Date of publication and mention of the grant of the patent: **23.07.2014**
(21) Application number: 06728690.6
(22) Date of filing: 07.03.2006
(51) Int. Cl.: A61K 38/00, A61P 1/16, A61P 9/00, A61P 9/10, A61P 11/00, A61P 13/12, A61P 43/00

(54) **METHOD FOR REGENERATING AND REPAIRING DAMAGED TISSUES USING ADRENOMEDULLIN**
VERFAHREN ZUR REGENERATION ODER REPARATUR VON GESCHÄDIGTEM GEWEBE MIT ADRENOMEDULIN
PROCEDE DE REGENERATION OU DE REPARATION D'UN TISSU ENDOMMAGE UTILISANT DE L'ADRENOMEDULLINE

(30) Priority: 07.03.2005 JP 2005062951
(43) Date of publication of application: 16.01.2008
(73) Proprietor: National Cerebral and Cardiovascular Center, Suita-shi Osaka 5658565 (JP); Hubit Genomix, Inc., Chuo-ku Tokyo 104-0045 (JP)
(72) Inventor: NAGAYA, Noritoshi, Suita-shi, Osaka, 5658565 (JP); MIYATAKE, Kunio, Kawachinagano-shi, Osaka, 5868521 (JP); KANGAWA, Kenji, Suita-shi, Osaka, 5658565 (JP); MURAKAMI, Shinsuke, Nara 633-0054 (JP)
(74) Representative: Bosch, Matthias
(86) International application number: PCT/JP2006/304321
(87) International publication number: WO 2006/095711

(56) References cited:
- WO-A-99/16406
- WO-A-2005/026741
- JP-A- 07 196 693
- JP-A- 2001 524 454
- JP-A- 2005 206 491
- NAGAYA N ET AL: "Adrenomedullin in the treatment of pulmonary hypertension" PEPTIDES, ELSEVIER, AMSTERDAM, vol. 25, no. 11, 1 November 2004 (2004-11-01), pages 2013-2018, XP004610743 ISSN: 0196-9781
- IWASE TAKASHI ET AL: "Adrenomedullin enhances angiogenic potency of bone marrow transplantation in a rat model of hindlimb ischemia." CIRCULATION 25 JAN 2005, vol. 111, no. 3, 25 January 2005 (2005-01-25), pages 356-362, XP002539896 ISSN: 1524-4539
- MIYASHITA K. ET AL.: 'Adrenomedullin provokes endothelial Akt activation and promotes vascular regeneration both in vitro and in vivo' FEBS LETTERS vol. 544, no. 1-3, 2003, pages 16 - 92, XP004427871
- MIYASHITA K. ET AL.: 'Clinical application of adrenomedullin to therapeutic angiogenesis' NIPPON RINSHO: JAPANESE JOURNAL OF CLINICAL MEDICINE vol. 62, no. SUPPL. 9, 2004, pages 307 - 313, XP003001560
- NAKAMURA R. ET AL.: 'Adrenomedullin administration immediately after myocardial infarction ameliorates progression of heart failure in rats' CIRCULATION vol. 110, no. 4, 2004, pages 426 - 431, XP003001561
- NAKAMURA R. ET AL.: 'Potential of adrenomedullin as a therapeutic tool for left ventricular remodeling after myocardial infarction' NIPPON RINSHO: JAPANESE JOURNAL OF CLINICAL MEDICINE vol. 62, no. SUPPL. 9, 2004, pages 302 - 306, XP003001562
- Satoshi KITAMURA, separate volume. "Igaku no Ayumi Kokyuki Shikkan - states of arts (Ver.3)", 1st edition, 1st print, Iyakushi Shuppan Kabushiki Kaisa, 1999, pages 345 to 347, COPD-Manshei Heisokusei Haishikkan. XP003001563

### SEQUENCE LISTING

<110> JAPAN AS REPRESENTED BY GENERAL DIRECTOR OF AGENCY OF NATIONAL CARDIOVASCULAR CENTER HUBIT GENOMIX, INC.
<120> Method of regenerating and/or restoring damaged tissue using adrenomedullin
<130> H2-A0502P
<150> JP 2005-62951
   <151> 2005-03-07
<160> 3
<170> PatentIn version 3.3
<210> 1
   <211> 1457
   <212> DNA
   <213> Homo sapiens
<220>
   <221> CDS
   <222> (165)..(722)
<400> 1
   ggcacgagct ggatagaaca gctcaagcct tgccacttcg ggcttctcac tgcagctggg     60
   cttggacttc ggagttttgc cattgccagt gggacgtctg agactttctc cttcaagtac    120
   ttggcagatc actctcttag cagggtctgc gcttcgcagc cggg atg aag ctg gtt     176
                                                    Met Lys Leu Val
                                                    1
   tcc gtc gcc ctg atg tac ctg ggt tcg ctc gcc ttc cta ggc gct gac      224
   Ser Val Ala Leu Met Tyr Leu Gly Ser Leu Ala Phe Leu Gly Ala Asp
   5                   10                  15                  20
   acc gct cgg ttg gat gtc gcg tcg gag ttt cga aag aag tgg aat aag      272
   Thr Ala Arg Leu Asp Val Ala Ser Glu Phe Arg Lys Lys Trp Asn Lys
                   25                  30                  35
   tgg gct ctg agt cgt ggg aag agg gaa ctg cgg atg tcc agc agc tac      320
   Trp Ala Leu Ser Arg Gly Lys Arg Glu Leu Arg Met Ser Ser Ser Tyr
               40                  45                  50
   ccc acc ggg ctc gct gac gtg aag gcc ggg cct gcc cag acc ctt att      368
   Pro Thr Gly Leu Ala Asp Val Lys Ala Gly Pro Ala Gln Thr Leu Ile
           55                  60                  65
   cgg ccc cag gac atg aag ggt gcc tct cga agc ccc gaa gac agc agt      416
   Arg Pro Gln Asp Met Lys Gly Ala Ser Arg Ser Pro Glu Asp Ser Ser
       70                  75                  80
   ccg gat gcc gcc cgc atc cga gtc aag cgc tac cgc cag agc atg aac      464
   Pro Asp Ala Ala Arg Ile Arg Val Lys Arg Tyr Arg Gln Ser Met Asn
   85                  90                  95                  100
   aac ttc cag ggc ctc cgg agc ttt ggc tgc cgc ttc ggg acg tgc acg      512
   Asn Phe Gln Gly Leu Arg Ser Phe Gly Cys Arg Phe Gly Thr Cys Thr
                   105                 110                 115
   gtg cag aag ctg gca cac cag atc tac cag ttc aca gat aag gac aag      560
   Val Gln Lys Leu Ala His Gln Ile Tyr Gln Phe Thr Asp Lys Asp Lys
               120                 125                 130
   gac aac gtc gcc ccc agg agc aag atc agc ccc cag ggc tac ggc cgc      608
   Asp Asn Val Ala Pro Arg Ser Lys Ile Ser Pro Gln Gly Tyr Gly Arg
           135                 140                 145
   cgg cgc cgg cgc tcc ctg ccc gag gcc ggc ccg ggt cgg act ctg gtg      656
   Arg Arg Arg Arg Ser Leu Pro Glu Ala Gly Pro Gly Arg Thr Leu Val
       150                 155                 160
   tct tct aag cca caa gca cac ggg gct cca gcc ccc ccg agt gga agt      704
   Ser Ser Lys Pro Gln Ala His Gly Ala Pro Ala Pro Pro Ser Gly Ser
   165                 170                 175                 180
   gct ccc cac ttt ctt tag gatttaggcg cccatggtac aaggaatagt              752
   Ala Pro His Phe Leu
                   185
   cgcgcaagca tcccgctggt gcctcccggg acgaaggact tcccgagcgg tgtggggacc     812
   gggctctgac agccctgcgg agaccctgag tccgggaggc accgtccggc ggcgagctct     872
   ggctttgcaa gggcccctcc ttctgggggc ttcgcttcct tagccttgct caggtgcaag     932
   tgccccaggg ggcggggtgc agaagaatcc gagtgtttgc caggcttaag gagaggagaa     992
   actgagaaat gaatgctgag acccccggag caggggtctg agccacagcc gtgctcgccc    1052
   acaaactgat ttctcacggc gtgtcacccc accagggcgc aagcctcact attacttgaa    1112
   ctttccaaaa cctaaagagg aaaagtgcaa tgcgtgttgt acatacagag gtaactatca    1172
   atatttaagt ttgttgctgt caagattttt tttgtaactt caaatataga gatatttttg    1232
   tacgttatat attgtattaa gggcatttta aaagcaatta tattgtcctc ccctatttta    1292
   agacgtgaat gtctcagcga ggtgtaaagt tgttcgccgc gtggaatgtg agtgtgtttg    1352
   tgtgcatgaa agagaaagac tgattacctc ctgtgtggaa gaaggaaaca ccgagtctct    1412
   gtataatcta tttacataaa atgggtgata tgcgaacagc aaacc                    1457
<210> 2
   <211> 185
   <212> PRT
   <213> Homo sapiens
<400> 2
   Met Lys Leu Val Ser Val Ala Leu Met Tyr Leu Gly Ser Leu Ala Phe
   1               5                   10                  15
   Leu Gly Ala Asp Thr Ala Arg Leu Asp Val Ala Ser Glu Phe Arg Lys
               20                  25                  30
   Lys Trp Asn Lys Trp Ala Leu Ser Arg Gly Lys Arg Glu Leu Arg Met
           35                  40                  45
   Ser Ser Ser Tyr Pro Thr Gly Leu Ala Asp Val Lys Ala Gly Pro Ala
       50                  55                  60
   Gln Thr Leu Ile Arg Pro Gln Asp Met Lys Gly Ala Ser Arg Ser Pro
   65                  70                  75                  80
   Glu Asp Ser Ser Pro Asp Ala Ala Arg Ile Arg Val Lys Arg Tyr Arg
                   85                  90                  95
   Gln Ser Met Asn Asn Phe Gln Gly Leu Arg Ser Phe Gly Cys Arg Phe
               100                 105                 110
   Gly Thr Cys Thr Val Gln Lys Leu Ala His Gln Ile Tyr Gln Phe Thr
           115                 120                 125
   Asp Lys Asp Lys Asp Asn Val Ala Pro Arg Ser Lys Ile Ser Pro Gln
       130                 135                 140
   Gly Tyr Gly Arg Arg Arg Arg Arg Ser Leu Pro Glu Ala Gly Pro Gly
   145                 150                 155                 160
   Arg Thr Leu Val Ser Ser Lys Pro Gln Ala His Gly Ala Pro Ala Pro
                   165                 170                 175
   Pro Ser Gly Ser Ala Pro His Phe Leu
               180                 185
<210> 3
   <211> 52
   <212> PRT
   <213> Homo sapiens
<400> 3
   Tyr Arg Gln Ser Met Asn Asn Phe Gln Gly Leu Arg Ser Phe Gly Cys
   1               5                   10                  15
   Arg Phe Gly Thr Cys Thr Val Gln Lys Leu Ala His Gln Ile Tyr Gln
               20                  25                  30
   Phe Thr Asp Lys Asp Lys Asp Asn Val Ala Pro Arg Ser Lys Ile Ser
           35                  40                  45
   Pro Gln Gly Tyr
       50

## Description

### Technical Field

The present invention relates to methods for regenerating or repairing damaged tissues using adrenomedullin, and pharmaceutical agents comprising adrenomedullin as an active ingredient for regenerating or repairing damaged tissues.

### Background Art

Pulmonary emphysema, an intractable lung disease, is globally the main cause of respiratory impairment and death, and is defined as an abnormal and permanent enlargement of the air space from terminal bronchiole to peripheral parts (Non-patent Documents 1 to 3). Since many mediators have partially overlapping actions in pulmonary emphysema, there are at present no effective treatments for preventing progression of this disease (Non-patent Document 4). One of the pathological changes in pulmonary emphysema, destruction of peripheral airways and alveolar walls, is so far thought to be irreversible. Therefore, present pharmacotherapies mainly focus on improving quality of life and exercise tolerance, and there are no effective pharmaceutical agents for suppressing the progression of inflammation or obstructive impairments of the respiratory tract.

Myocardial infarction is a condition in which cardiac muscles are necrosed as a result of coronary artery obstruction, and old myocardial infarction is an important cause of chronic heart failure. For chronic heart failure, pharmacotherapies aiming at preventing relapse of arrhythmia or myocardial infarction, progression of arteriosclerosis and the like are performed, but there are no effective treatments for recovering necrotized cardiac muscles. Trials have begun in recent years to improve cardiac functions by recruiting bone marrow cells to damaged sites in cardiac muscles using granulocyte colony-stimulating factor (G-CSF), and thereby promoting the regeneration of blood vessels and cardiac muscles; however, these cells have not been established yet as a therapeutic agent due to problems of side effects and the like.

It is important to recover the functions by regenerating or repairing damaged tissues in intractable cardiopulmonary diseases such as pulmonary emphysema and myocardial infarction; however, no effective treatments have been established as of now.
[Non-patent Document 1] Lopez AD, Murray CC. The global burden of disease, 1990-2020. Nat Med 1998;4:1241-1243.
[Non-patent Document 2] Michaud CM, Murray CJ, Bloom BR. Burden of disease--implications for future research. JAMA 2001;285:535-539.
[Non-patent Document 3] American Thoracic Society. Standards for the diagnosis and care of patients with chronic obstructive pulmonary disease. Am J Respir Crit Care Med 1995;152:577-S121.
[Non-patent Document 4] Barnes PJ. Chronic obstructive pulmonary disease. N Engl J Med 2000;343:269-280.
[Non-patent Document 5] Ishizawa K, Kubo H, Yamada M, Kobayashi S, Numasaki M, Ueda S, Suzuki T, Sasaki H. Bone marrow-derived cells contribute to lung regeneration after elastase-induced pulmonary emphysema. FEBS Lett 2004;556:249-252.
[Non-patent Document 6] Yamada M, Kubo H, Kobayashi S, Ishizawa K, Numasaki M, Ueda S, Suzuki T, Sasaki H. Bone marrow-derived progenitor cells are important for lung repair after lipopolysaccharide-induced lung injury. J Immunol 2004;172:1266-1272.
[Non-patent Document 7] Kitamura K, Kangawa K, Kawamoto M, Ichiki Y, Nakamura S, Matsuo H, Eto T. Adrenomedullin: a novel hypotensive peptide isolated from human pheochromocytoma. Biochem Biophys Res Commun 1993;192:553-560.
[Non-patent Document 8] Ichiki Y, Kitamura K, Kangawa K, Kawamoto M, Matsuo H, Eto T. Distribution and characterization of immunoreactive adrenomedullin in human tissue and plasma. FEBS Lett 1994;338:6-10.
[Non-patent Document 9] Sakata J, Shimokubo T, Kitamura K, Nishizono M, Iehiki Y, Kangawa K, Matsuo H, Eto T. Distribution and characterization of immunoreactive rat adrenomedullin in tissue and plasma. FEBS Lett 1994;352:105-108.
[Non-patent Document 10] Martinez A, Miller MJ, Catt KJ, Cuttitta F. Adrenomedullin receptor expression in human lung and in pulmonary tumors. J Histochem Cytochem 1997;45:159-164.
[Non-patent Document 11] Nishimatsu H, Suzuki E, Nagata D, Moriyama N, Satonaka H, Walsh K, Sata M, Kangawa K, Matsuo H, Goto A, et al. Adrenomedullin induces endothelium-dependent vasorelaxation via the phosphatidylinositol 3-kinase/Akt-dependent pathway in rat aorta. Circ Res 2001;89 (1):63-70.
[Non-patent Document 12] Shiojima I, Walsh K. Role of Akt signaling in vascular homeostasis and angiogenesis. Circ Res 2002;90:1243-1250.
[Non-patent Document 13] Kim W, Moon SO, Sung MJ, Kim SH, Lee S, So JN, Park SK. Angiogenic role of adrenomedullin through activation of Akt, mitogen-activated protein kinase, and focal adhesion kinase in endothelial cells. FASEB J 2003;17:1937-1939.
[Non-patent Document 14] Tokunaga N, Nagaya N, Shirai M, Tanaka E, Ishibashi-Ueda H, Harada-Shiba M, Kanda M, Ito T, Shimizu W, Tabata Y, et al. Adrenomedullin gene transfer induces therapeutic angiogenesis in a rabbit model of chronic hind limb ischemia: benefits of a novel nonviral vector, gelatin. Circulation 2004;109:526-531
[Non-patent Document 15] Abe M, Sata M, Nishimatsu H, Nagata D, Suzuki E, Terauchi Y, Kadowaki T, Minamino N, Kangawa K, Matsuo H, Hirata Y, Nagai R. Adrenomedullin augments collateral development in response to acute ischemia, Biochem. Biophys. Res. Commun. 2003;306(1):10-15
[Non-patent Document 16] Nagaya N, Kyotani S, Uematsu M, Ueno K, Oya H, Nakanishi N, Shirai M, Mori H, Miyatake K, Kangawa K. Effects of Adrenomedullin Inhalation on Hemodynamics and Exercise Capacity in Patients With Idiopathic Pulmonary Arterial Hypertension. Circulation, 2004;109(3):351-356
[patent Document 1] Japanese Patent Application Kokai Publication No. (JP-A) H07-196693 (unexamined, published Japanese patent application)
[Patent Document 2] WO011027310
[Patent Document 3] WO00/078339
[Patent Document 4] WO00/078338
[Patent Document 5] JP-A 2003-300899

### Disclosure of the Invention

### [Problems to be Solved by the Invention]

Adrenomedullin (hereinafter, sometimes indicated as "AM") is a powerful vasodilating peptide, and was first isolated from human pheochromocytoma (Non-patent Document 7). Later, immunoreactive AM has been detected in various tissues including the lung (Non-patent Documents 8 and 9). The AM receptor is highly expressed in basal cells of the respiratory epithelium and type II alveolar cells. Each of these cells participates in regeneration of the lung epithelium (Non-patent Document 10). In recent studies, it has been shown that AM activates the phosphatidylinositol 3-kinase/Akt-dependent pathway, which is considered to regulate many important stages of cell proliferation, in vascular endothelial cells (Non-patent Documents 11 to 14). In view of these findings, AM is highly likely to play an important role in the regulation of lung homeostasis. However, it is still unknown whether AM participates in lung regeneration, or whether AM promotes regeneration of alveoli and lung vasculature, thereby improving lung structure and functions.

In recent years, trials have been made to regenerate organs by recruiting stem cells and precursor cells contained in the bone marrow to peripheral tissues using cytokines and chemokines, and then differentiating them into blood vessels, cardiac muscles, or alveoli at lesion sites (Non-patent Documents 5 and 6); however, they have not been established as treatments. Since adrenomedullin recruits bone marrow cells to the periphery and regenerates or repairs damaged tissues, it can serve as a new therapeutic agent for various organ damages caused by brain, liver, and renal diseases and such, as well as intractable cardiopulmonary diseases.

The present invention was achieved in view of such circumstances. An objective of the present invention is to provide methods for regenerating or repairing damaged tissues using adrenomedullin. Moreover, another objective is to provide pharmaceutical agents comprising adrenomedullin as an active ingredient for regenerating or repairing damaged tissues.

### [Means for Solving the Problems]

In order to solve the above problems, the present inventors first administered adrenomedullin or physiological saline at random to C57BL/6 mice for five days, and counted the numbers of mononuclear cells and Sca-1-positive cells in blood. As a result, it was shown that adrenomedullin increased the numbers of mononuclear cells and Sca-1-positive cells in blood.

Further, after intratracheal injection of swine pancreatic elastase or physiological saline, the present inventors randomized the mice, performed continuous infusion of adrenomedullin or physiological saline for 14 days, and conducted functional and histological analyses 28 days after the administration. On day 28 after elastase injection, air space enlargement accompanied by destruction of alveolar walls was observed. However, it was found that the increase in lung volume, static lung compliance, and average alveolar diameter of the elastase-treated mice were significantly suppressed by adrenomedullin injection. In addition, it was shown that adrenomedullin significantly increases the number of bone marrow-derived cells in the elastase-administered lungs, and a portion of these cells were positive for cytokeratin (an epithelial cell marker) or von Willebrand factor (an endothelial cell marker).

Moreover, using a rat model of acute myocardial infarction, the present inventors examined the dynamics (distribution) of bone marrow-derived cells and vascular endothelial cells and the effects of adrenomedullin administration on revascularization in ischemic cardiac muscles. The result clearly showed that after acute myocardial infarction, bone marrow-derived cells reached the ischemic cardiac muscles and accumulated there to form blood vessels. It was also found that adrenomedullin administration after acute myocardial infarction significantly increased the revascularization by the bone marrow cells. In addition, a small portion of the bone marrow-derived cells were shown to settle in the ischemic cardiac muscles and express cardiac muscle cell markers.

From the above results, the present inventors found that administration of adrenomedullin, a circulation control peptide, increased the number of bone marrow cells migrating and settling to damaged tissue and that the recruited bone marrow cells regenerated blood vessels, alveoli, or cardiac muscle cells at lesion sites. Consequently, in small animal models of myocardial infarction and pulmonary emphysema, reduction of infarction size and suppression of alveolar diameter expansion as well as functional improvement were further shown.

Specifically, the present inventors succeeded in developing methods for regenerating or repairing damaged tissues in subjects by administering adrenomedullin to the subjects, and thereby completed the present invention.

More specifically, the present invention provides the following [1] to [22]:
[1] a method for regenerating or repairing a damaged tissue in a subject, wherein the method comprises the step of administering adrenomedullin to the subject;
[2] a method for promoting migration and settlement of bone marrow-derived cells into a damaged tissue in a subject, wherein the method comprises the step of administering adrenomedullin to the subject;
[3] a method for directing bone marrow-derived cells into the peripheral blood of a damaged tissue in a subject, wherein the method comprises the step of administering adrenomedullin to the subject;
[4] a method for increasing bone marrow-derived cells contained in the peripheral blood of a damaged tissue in a subject, wherein the method comprises the step of administering adrenomedullin to the subject;
[5] the method of any one of [2] to [4], wherein the bone marrow-derived cell comprises a stem cell or precursor cell;
[6] a method for newly generating or regenerating a peripheral vessel or a cell in a damaged tissue in a subject, wherein the method comprises the step of administering adrenomedullin to the subject;
[7] the method of any one of [1] to [6], wherein the damaged tissue in the subject is a damaged tissue in any one of an intractable cardiopulmonary disease, peripheral vascular disease, renal disease, liver disease, and cerebrovascular disease;
[8] the method of any one of [1] to [6], wherein the damaged tissue in the subject is a damaged tissue in pulmonary emphysema;
[9] the method of any one of [1] to [6], wherein the damaged tissue in the subject is a damaged tissue in an alveolus or lung blood vessel;
[10] the method of any one of [1] to [6], wherein the damaged tissue in the subject is a damaged tissue in any one of myocardial infarction, myocarditis, and myocardosis;
[11] a method for decreasing lung volume, static lung compliance, or average alveolar diameter, wherein the method comprises the step of administering adrenomedullin to a subject;
[12] a pharmaceutical agent for regenerating or repairing a damaged tissue, wherein the agent comprises adrenomedullin as an active ingredient;
[13] a pharmaceutical agent for promoting migration and settlement of bone marrow-derived cells into a damaged tissue, wherein the agent comprises adrenomedullin as an active ingredient;
[14] a pharmaceutical agent for directing bone marrow-derived cells into the peripheral blood of a damaged tissue, wherein the agent comprises adrenomedullin as an active ingredient;
[15] a pharmaceutical agent for increasing bone marrow-derived cells contained in the peripheral blood of a damaged tissue, wherein the agent comprises adrenomedullin as an active ingredient;
[16] the pharmaceutical agent of any one of [13] to [15], wherein the bone marrow-derived cell comprises a stem cell or precursor cell;
[17] a pharmaceutical agent for newly generating or regenerating a peripheral vessel or a cell in a damaged tissue, wherein the agent comprises adrenomedullin as an active ingredient;
[18] the pharmaceutical agent of any one of [12] to [17], wherein the damaged tissue is in any one of an intractable cardiopulmonary disease, peripheral vascular disease, renal disease, liver disease, and cerebrovascular disease;
[19] the pharmaceutical agent of any one of [12] to [17], wherein the damaged tissue is in pulmonary emphysema;
[20] the pharmaceutical agent of any one of [12] to [17], wherein the damaged tissue is in an alveolus or lung blood vessel;
[21] the pharmaceutical agent of any one of [12] to [17], wherein the damaged tissue is in any one of myocardial infarction, myocarditis, and myocardosis; and
[22] a pharmaceutical agent for decreasing lung volume, static lung compliance, or average alveolar diameter, wherein the agent comprises adrenomedullin as an active ingredient.

### Brief Description of the Drawings

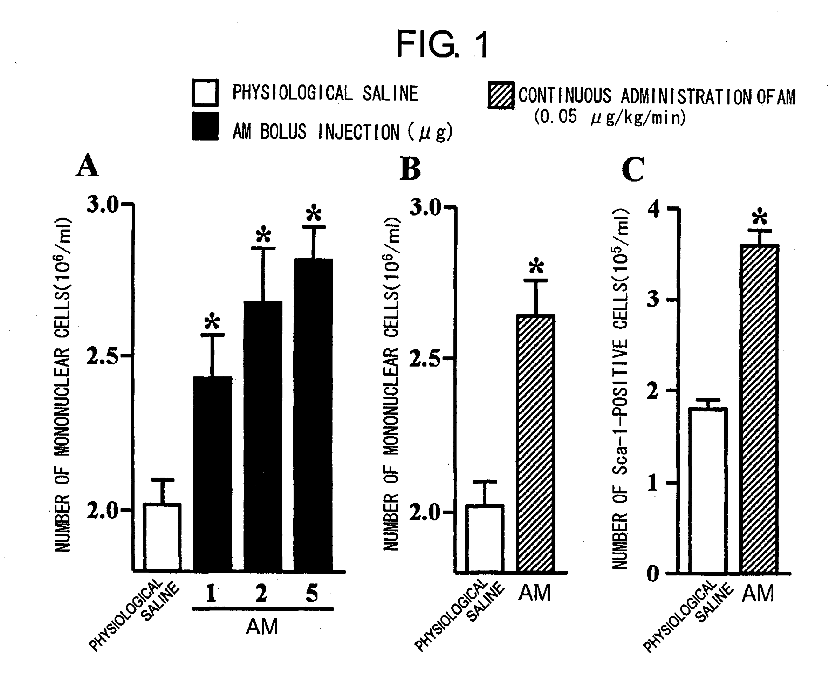

Fig. 1 (A) is a graph showing the effect of adrenomedullin (AM) bolus injection on the number of peripheral blood mononuclear cells; Fig. 1 (B) is a graph showing the effect of AM continuous administration on the number of peripheral blood mononuclear cells; and Fig. 1 (C) is a graph showing the effect of continuous AM administration on the number of Sca-1-positive cells in the lung. Data are averages ± SEM. * P < 0.05, compared with the physiological saline group.

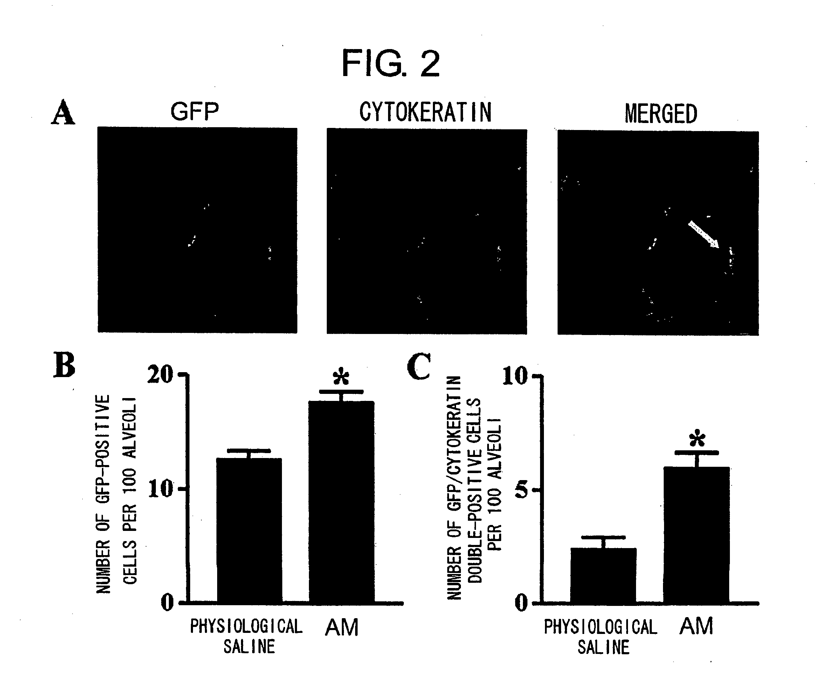

Fig. 2 (A) represents photographs showing representative examples of bone marrow cell differentiation into an epithelial cell line. The left photograph indicates fluorescence of green fluorescence protein (GFP). The middle indicates fluorescence of cytokeratin, an epithelial cell marker. The right represents a combination of the individual fluorescences, and arrows show fluorescent sites of either green fluorescence protein or cytokeratin which is an epithelial cell marker. Magnification: x 400.
Fig. 2 (B) shows a semiquantitative analysis of cell migration. The number of GFP-positive cells present within alveolar walls in the AM group was significantly larger than that in the physiological saline group.
Fig. 2 (C) shows a semiquantitative analysis of epithelial differentiation. The number of GFP/cytokeratin double-positive cells in the AM group was significantly larger than that in the physiological saline group.

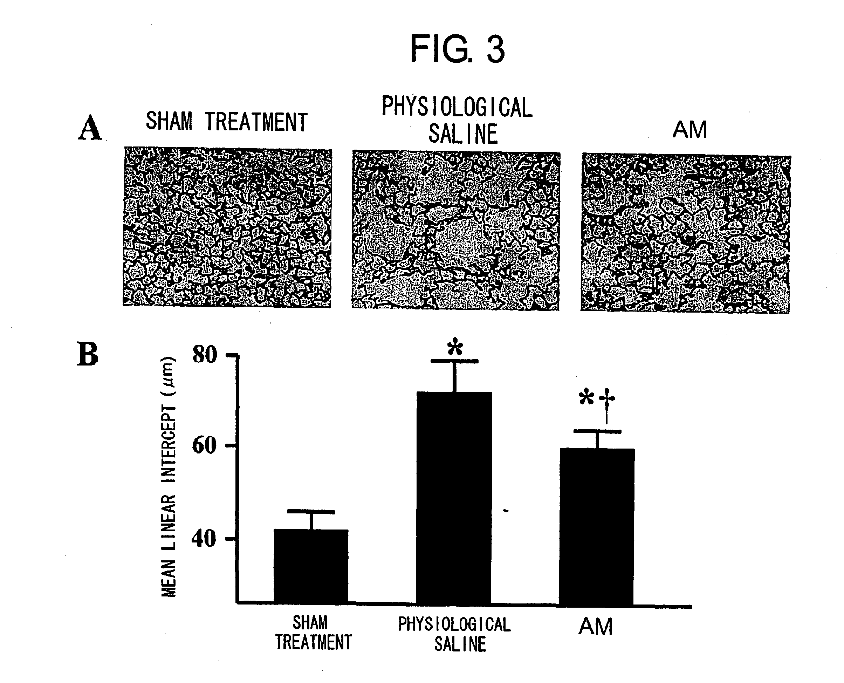

Fig. 3 (A) presents photographs showing representative examples of lung tissues stained with hematoxylin-eosin. Intratracheal injection of elastase induced development of air space enlargement accompanied by alveolar wall destruction in mouse lungs (the physiological saline group). Emphysematous changes induced by elastase were decreased by AM injection (the AM group). Magnification: x 100.
Fig. 3 (B) shows a semiquantitative analysis of lung tissues by using the average alveolar diameter, a morphometric parameter of pulmonary emphysema. Data are averages ± SEM. * P < 0.05, compared with the sham treatment group; † P < 0.05, compared with the physiological saline group.

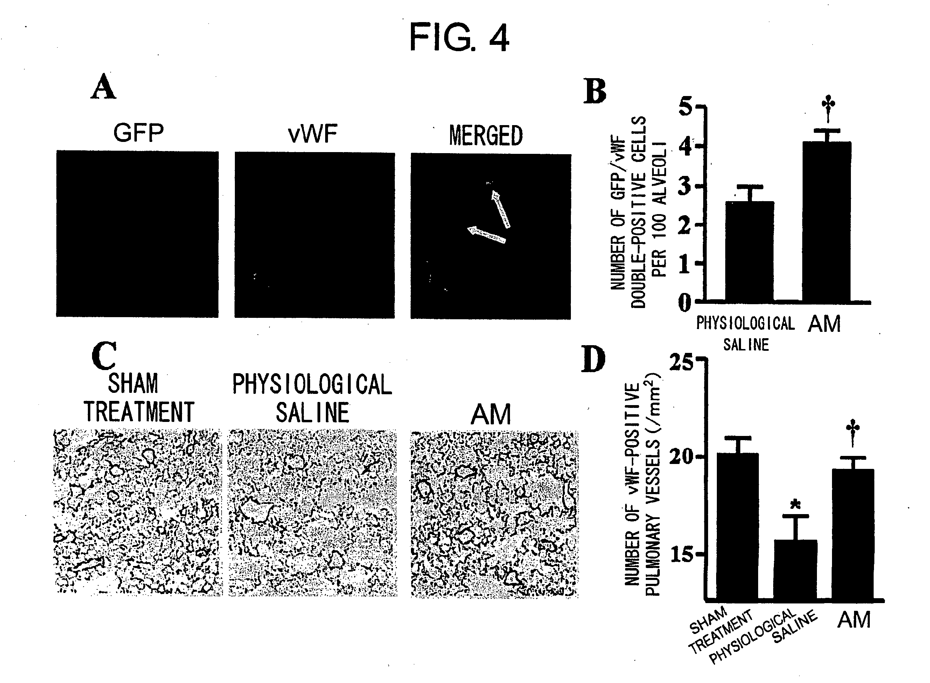

Fig. 4 (A) presents photographs showing representative examples of bone marrow cell differentiation into an endothelial cell line. The left photograph indicates fluorescence of green fluorescence protein (GFP). The middle indicates fluorescence of von Willebrand factor (vWF), an endothelial cell marker. The right displays a combination of the individual fluorescences, and arrows show fluorescent sites of either green fluorescence protein or von Willebrand factor (vWF) which is an endothelial cell marker. Magnification: x 400.
Fig. 4(B) shows a semiquantitative analysis of endothelial differentiation. The number of GFP/vWF double-positive cells in the AM group was significantly larger than that in the physiological saline group.
Fig. 4 (C) shows photographs of vWF immunohistochemical analysis. The observed frequency of vWF-positive blood vessels was lower in the elastase-injected and physiological saline-administered mice, but this frequency was increased by the AM injection. Magnification: x 100.
Fig. 4 (D) shows by a semiquantitative analysis that the number of vWF-positive blood vessels was decreased on day 28 after elastase injection (the physiological saline group). However, the number of the vWF positive-blood vessels in the elastase-administered lungs was significantly increased by AM injection (the AM group). Data are averages ± SEM. * P < 0.05, compared with the sham treatment group; † P < 0.05, compared with the physiological saline group.

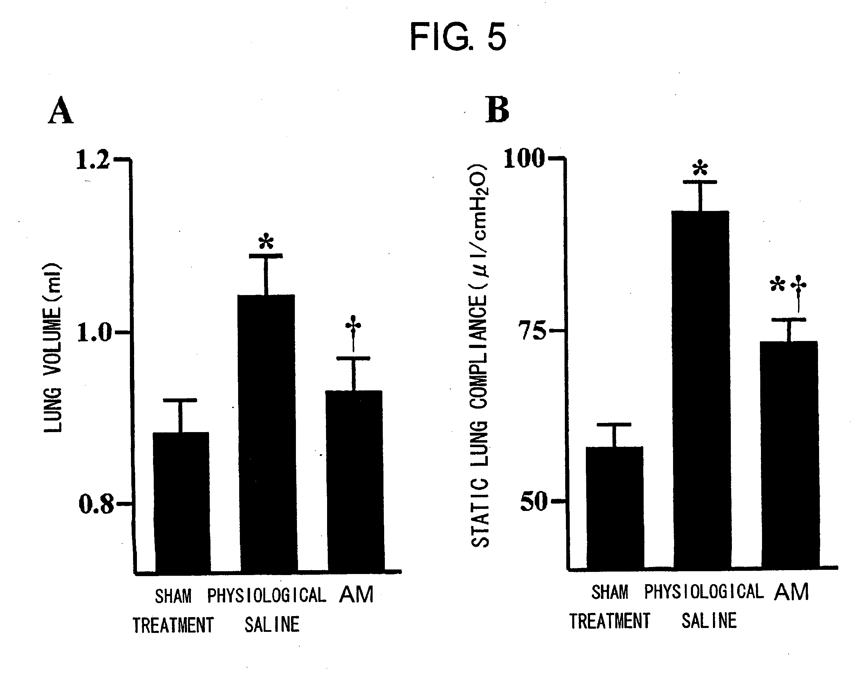

Fig. 5 shows the effects of AM on lung volume (A) and static lung compliance (B) of the mice on day 28 after elastase injection. By the elastase injection, the lung volume and compliance of the mice were significantly increased (the physiological saline group). These changes in the elastase-treated mice were significantly suppressed by AM injection (the AM group). Data are averages ± SEM. * P < 0.05, compared with the sham treatment group; †P < 0.05, compared with the physiological saline group.

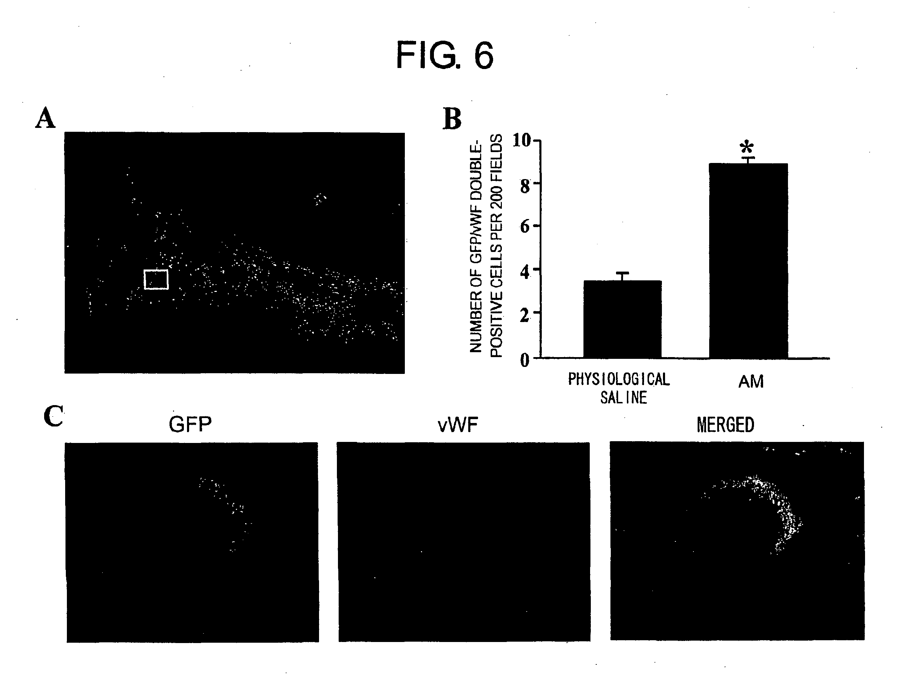

Fig. 6 presents photographs and a graph showing the dynamics of bone marrow-derived cells and vascular endothelial cells in ischemic cardiac muscles after acute myocardial infarction. Fig. 6 (A) is a photograph showing the distributions of bone marrow-derived cells (confirmed by GFP expression) and vascular endothelial cells (confirmed by vWF expression) after acute myocardial infarction. Fig. 6 (B) shows the total numbers of bone marrow-derived cells and vascular endothelial cells in the AM group and the control group. Data are averages ± SEM. *P < 0.05, compared with the control group. Fig. 6 (C) presents photographs showing the distributions of bone marrow-derived cells (confirmed by GFP expression) and vascular endothelial cells (confirmed by vWF expression) in a certain part of the ischemic cardiac muscle.

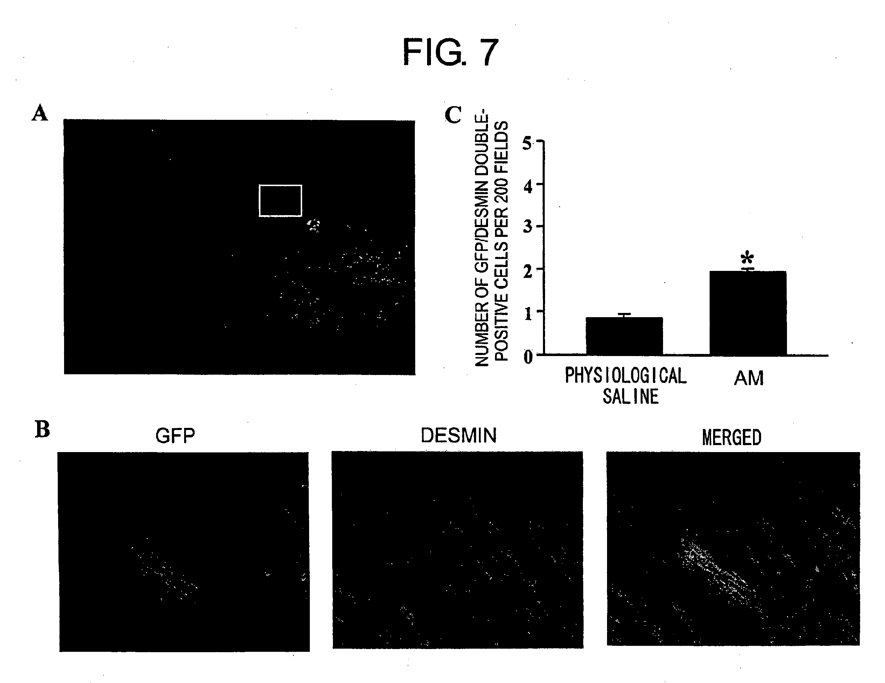

Fig. 7 presents photographs and a graph showing the dynamics of bone marrow-derived cells and cardiac muscle cells in ischemic cardiac muscles after acute myocardial infarction. Fig. 7 (A) is a photograph showing the distributions of bone marrow-derived cells (confirmed by GFP expression) and cardiac muscle cells (confirmed by desmin expression) after acute myocardial infarction. Fig. 7 (B) presents photographs showing the distributions of bone marrow-derived cells (confirmed by GFP expression) and cardiac muscle cells (confirmed by desmin manifestation) in a certain part of ischemic cardiac muscle. A group of cardiac muscle cells simultaneously expressing GFP and desmin are observed, which cells have been regenerated or are being regenerated from bone marrow-derived cells. Fig. 7 (C) is a graph showing the total amounts of bone marrow-derived cells and cardiac muscle cells in the AM group and in the control group. Data are averages ± SEM. *P < 0.05, compared with the control group.

### Best Mode for Carrying Out the Invention

The present invention relates to methods for regenerating or repairing damaged tissues in subjects, comprising a step of administering adrenomedullin to the subjects.

In the present invention, the term "adrenomedullin" or "AM" refers to a bioactive peptide with a powerful antihypertensive effect discovered in a human pheochromocytoma tissue extract using the increase of cAMP activity in thrombocytes as an indicator. The nucleotide sequence of an "adrenomedullin" precursor cDNA is shown in SEQ ID NO: 1, and the amino acid sequence of a protein encoded by the cDNA is shown in SEQ ID NO: 2.

"Adrenomedullin" precursors in the present invention can include naturally-occurring proteins which comprise an amino acid sequence with one or more amino acid substitutions, deletions, insertions, and/or additions in the amino acid sequence of SEQ ID NO: 2, and which are functionally equivalent to the protein consisting of the amino acid sequence of SEQ ID NO: 2. Moreover, the "adrenomedullin" precursors can include proteins which are encoded by naturally-occurring DNAs that hybridize under stringent conditions with DNAs comprising the nucleotide sequence of SEQ ID NO: 1, and which are functionally equivalent to the protein consisting of the amino acid sequence of SEQ ID NO: 2.

Active "adrenomedullin" is part of the "adrenomedullin" precursors, and comprises the amino acid sequence of SEQ ID NO: 3. Adrenomedullin used in the present invention preferably comprises the amino acid sequence of SEQ ID NO: 3, but is not limited thereto. It can include naturally-occurring proteins which comprise an amino acid sequences with one or more amino acid substitutions deletions, insertions, and/or additions in the amino acid sequence of SEQ ID NO: 3, and which are functionally equivalent to the protein consisting of the amino acid sequence of SEQ ID NO: 3. The active "adrenomedullin" can also include proteins which are encoded by naturally-occurring DNAs that hybridize under stringent conditions with the DNA corresponding to positions 447 to 602 in the nucleotide sequence of SEQ ID NO: 1, and which are functionally equivalent to the protein consisting of the amino acid sequence of SEQ ID NO: 3.

In the present invention, the number of mutated amino acids is not particularly limited, but is usually 30 amino acids or less, preferably 15 amino acids or less, and more preferably 5 amino acids or less (for example, 3 amino acids or less). Amino acid residues are preferably mutated to amino acids that conserve amino acid side chain properties. For example, examples of amino acids with amino acid side chain properties include: hydrophobic amino acids (A, I, L, M, F, P, W, Y, and V), hydrophilic amino acids (R, D, N, C, E, Q, G, H, K, S, and T), amino acids with aliphatic side chains (G, A, V, L, I, and P), amino acids with hydroxyl-containing side chains (S, T, and Y), amino acids with sulfur-containing side chains (C and M), amino acids with carboxylic acid- and amide-containing side chains (D, N, E, and Q), amino acids with base-containing side chains (R, K, and H), and amino acids with aromatic-containing side chains (H, F, Y, and W) (amino acids are represented by one-letter codes in the parentheses). It is known to those skilled in the art that a polypeptide that comprises an amino acid sequence modified by a deletion, addition, and/or substitution with another amino acid of one or more amino acid residues maintains its biological activity.

In the present invention, the term "functionally equivalent" means that the protein has biological and biochemical functions equivalent to those of a protein of interest. In the present invention, biological and biochemical functions of a protein of interest can include the function to regenerate or repair a damaged tissue in a subject; the function to promote migration and settlement of mononuclear cells into the damaged tissue; the function to direct mononuclear cells into peripheral blood of the damaged tissue in the subject; the function to increase mononuclear cells contained in the peripheral blood of the damaged tissue; the function to generate or regenerate peripheral vessels of the damaged tissue; and the function to decrease the lung volume, static lung compliance, or average alveolar diameter. Methods for measuring such functions can be, for example, methods described in the Examples, but are not limited thereto. Biological properties comprise expression site specificity and expression level.

Methods well known to those skilled in the art for preparing DNAs that encode "proteins which are functionally equivalent" to target proteins include methods that utilize hybridization or polymerase chain reaction (PCR) techniques. Namely, those skilled in the art can routinely isolate DNAs that are highly homologous to "adrenomedullin" by using the nucleotide sequence of "adrenomedullin" (SEQ ID NO: 1) or a part thereof as a probe, or by using oligonucleotides that specifically hybridize with the nucleotide sequence of "adrenomedullin" (SEQ ID NO: 1) as primers. DNAs encoding proteins that are functionally equivalent to "adrenomedullin" and which can be isolated by hybridization or PCR techniques are also comprised in the DNAs of the present invention.

In order to isolate such DNAs, the hybridization reaction is preferably performed under stringent conditions. In the present invention, "stringent hybridization condition" refers to conditions of 6M urea, 0.4% SDS, and 0.5x SSC, or hybridization conditions with an equivalent stringency to these conditions. DNAs with higher homology may be isolated by using conditions with higher stringency, for example, conditions of 6M urea, 0.4% SDS, and 0.1x SSC. The DNAs thus isolated are considered to have high homology with the amino acid sequence of the target protein at the amino acid level. High homology refers to a sequence identity of at least 50% or more, more preferably 70% or more, still more preferably 90% or more (for example, 95%, 96%, 97%, 98%, 99% or more) across the whole amino acid sequence. The identity of amino acid sequences and nucleotide sequences can be determined using the BLAST algorithm by Karlin and Altschul. Programs called BLASTN and BLASTX have been developed based on the BLAST algorithm. When analyzing nucleotide sequences using BLASTN, parameters are set, for example, to score = 100 and wordlength = 12. When analyzing amino acid sequences using BLASTX, parameters are set to, for example, score = 50 and wordlength = 3. When using BLAST and Gapped BLAST programs, the default parameters of each program are used. The specific procedures of these analyzing methods are known.

In the present invention, the term "subject" means an organism to be treated by the methods of the present invention, part of the body of the organism (for example, organs such as brain, lung, heart, liver, and kidney, and blood vessels or tissues of an organ), or a part excised or excreted from the organism. The organism can be any animal (for example, humans, domestic animals, and wild animals) without particular limitation, but preferably comprises mammals, and more preferably humans. In the present invention, the tem "subject" can also refer to a tissue that has received damage itself (a damaged tissue).

In the present invention, the term "administer" comprises administering orally or parenterally.

Oral administrations include administration in the form of an oral agent, and the oral agent can be in a dosage form such as granule, powder, tablet, capsule, solution, emulsion, or suspension.

Parenteral administrations include administration in the form of an injection and the injection can be a subcutaneous injection, an intramuscular injection, or an intraperitoneal injection. These injections may be locally administered to target a part (such as a tissue in an organ) of the body of a subject organism, or may be administered to the blood vessel to circulate the adrenomedullin of the present invention throughout the whole organism. The injections may also be administered to two or more target sites simultaneously. In addition, the effects of the methods of the present invention can be achieved, by introducing into a living body a gene encoding the adrenomedullin to be administered using gene therapy methods. There are known methods for introducing into a living body genes encoding proteins that produce the effects of the methods of the present invention and then expressing them. Moreover, the adrenomedullin of the present invention can be locally administered to the region to be treated. For example, it can be administered by local injection during an operation, by use of a catheter, or by targeted gene delivery of a sequence encoding a therapeutic agent.

The dosage is different depending on the age, sex, weight and symptoms of the patient, the therapeutic effect, administration method, treatment time, and the type of active ingredients comprised in the pharmaceutical composition or the like, but usually one dose can be in the range of 0.1 mg to 500 mg, and preferably in the range of 0.5 mg to 20 mg per adult. However, since the dosage varies according to various conditions, sometimes a lower dosage than the above-mentioned dose may be enough, or a dosage exceeding the above-mentioned range may be required.

When the adrenomedullin of the present invention is administered to a part excised or excreted from an organism, it may be brought into "contact" with the part of the organism.

In the present invention, "contacting" is performed depending on the condition of the organism. For example, contacting comprises applying the adrenomedullin to a part of the organism, or adding the adrenomedullin to a fragment of the organism or the like, but it is not limited thereto.

When performing the methods of the present invention, the adrenomedullin of the present invention may be administrated as part of a pharmaceutical composition together with at least one known chemotherapeutic agent. In one embodiment, the adrenomedullin of the present invention and known chemotherapeutic agents may be administered at practically the same time.

It is also possible to administer the adrenomedullin of the present invention to a part excised or excreted from an organism, and then return the part to the organism from which it was excise or excreted or to other organisms.

In the present invention, various known methods can be used for inoculating injections. Preferable inoculation methods are subcutaneous injection, intramuscular injection, percutaneous inoculation and the like, but are not limited to thereto.

Suitable inoculation methods are determined in view of the type of agent, the type of subject to be inoculated or the like. Containers can be vials or pre-filled syringe products. If needed, the agents may be solutions or powdered products made by lyophilization and the like. The injections may be for single inoculation or multiple inoculations. The dosage varies depending on the type, weight, and age of the subject to be inoculated and the administration method, but an appropriate dosage can be suitably selected by those skilled in the art.

The term "damaged tissue" in the present invention can refer to a tissue that has received damage for a certain reason. When an external or internal stimulus is applied to a normal cell, usually it does not immediately lead to cell damage and an adaptation phenomenon occurs. However, if the external or internal stimulus exceeds adaptable range, morphological changes accompanying the stimulus, such as degeneration and necrosis, may take place; and such phenomena are defined as "damages" in the present invention.

In the present invention, the term "degeneration" in a damaged tissue refers to a change in a cell or tissue that is universally recognized as reversible cell damage. Some of the substances accumulated at the time of degeneration are normally present in a living body, and some are pathological substances not normally present. Degeneration is referred to as protein denaturation or fatty degeneration depending on the accumulated substance. Degenerate states include mucous degeneration, amyloid degeneration, degeneration as a result of α-1 antitrypsin deficiency, degeneration in uric acid metabolism disorders, hyaline droplet degeneration, dropsy-like degeneration, vacuolar degeneration, fibrinoid degeneration, and cornification.

In the present invention, the term "necrosis" refers to an irreversible change in cells or tissues. Necrotic states include coagulation necrosis (circulatory failure), colliquative necrosis, gangrene (a state in which bacterial infection is combined with necrosis), and apoptosis (physiological death occurred in tissues, not pathological death from cell damage).

In the present invention, the "damaged tissue in a subject" includes damaged tissues in diseases of the cranial-nerve system, endocrine system, circulatory organs, respiratory organs, digestive organs, urinary organs, skin and such.

The above-mentioned diseases include myocardial infarction, arrhythmia, arteriosclerosis, vasospasm, ischemic recirculation failure, pneumonia, infectious diseases, fibroid lung (adverse reaction of anticancer agents), ARDS, paraquat intoxication, smoking disorder, pulmonary emphysema, hyperoxia therapy, influenza, cerebral edema, cerebral infarction, cerebral hemorrhage, epilepsy, cerebrovascular spasm, Parkinson's disease, dysautonomia (Reilly phenomenon), late-onset neuropathy, spinal cord injury, neurogenic pulmonary edema, acute gastric mucosal disorder, gastric ulcer, ulcerative colitis, Crohn's disease, Behcet's disease, pneumonia, liver cirrhosis, drug-induced hepatopathy, pathology of liver transplantation, various types of clinical conditions of jaundice, pancreatitis, glomerulonephritis, hemolytic renal disorder, drug-induced renal disorder, burn, solar dermatitis, atopic dermatitis, skin ulcer, retinal degeneration, cataract, and corneal tumor. Preferably, the diseases include intractable cardiopulmonary diseases, peripheral vascular diseases, renal diseases, liver diseases, and cerebrovascular diseases, and more preferably, pulmonary emphysema, myocardial infarction, myocarditis, and myocardosis.

More specifically, the "damaged tissue in a subject" of the present invention includes damaged tissues in alveoli and pulmonary blood vessels. In addition, preferable examples include damaged tissues in cardiac muscles and heart blood vessels.

The term "regenerate or repair" in the present invention refers to returning the above-mentioned damaged tissue to the state before degeneration, or newly generating a damaged tissue that has necrotised. For example, generating or regenerating peripheral blood vessels of damaged tissues in a subject is also encompassed in the "regeneration or repair" of the present invention.

The period of regeneration or repair of a damaged tissue is not particularly limited, and may be temporary or for a fixed period of time. Moreover, although tissue regeneration or repair may be partial, it is preferable that regeneration or repair progresses fully, that is, until the tissues are recovered completely to their original condition.

More specifically, examples of "regeneration or repair" include repair of degenerated peripheral vessels, regeneration of new blood vessels to replace necrotized peripheral vessels, and regeneration of alveolar epithelia in alveoli or pulmonary blood vessels in a pulmonary emphysema condition. Consequently, when the lung volume, static lung compliance, or average alveolar diameter is decreased, it can be interpreted that the lung tissue has been repaired or regenerated.

The present invention relates to methods for promoting migration and settlement of mononuclear cells into damaged tissues in a subject, comprising the step of administering adrenomedullin to the subject.

In the present invention, the term "mononuclear cell" means a leukocyte comprising a nearly circular nucleus. It is a generic term for lymphocyte and monocyte, and includes cells derived from the bone marrow, the cord blood and the like. In the present invention, a more preferable example of "mononuclear cell" can include "bone marrow derived cell". In the present invention, "bone marrow-derived cell" refers to cells derived from the bone marrow, including stem cells and precursor cells.

Bone marrow-derived cells can be lymphocytes (B cells, helper T cells, suppressor T cells, killer T cells) and precursors thereof, monocytes (macrophages) and precursors thereof, or precursor cells of blood vessel-forming cells. However, in the present invention, they can be more preferably stem cells or precursor cells of various cells. In the present invention, preferable examples of precursor cells include vascular endothelial precursor cells and cardiac muscle precursor cells.

"Promote cell migration and settlement" as used herein means that the migration and settlement of cells, which are important for mechanisms such as angiogenesis, are further increased. The period during which cell migration and settlement are promoted is not particularly limited, and may be temporary or for a certain period of time. The meaning of "promote" in the present invention includes cases in which the number of cells that migrate and settle increases temporarily, or repeatedly increases and decreases within a certain period and is increased at the end.

In the present invention, "promote cell migration and settlement" may also mean that adrenomedullin induces the expression of adhesion factors such as inflammatory cytokines and chemokines, and thereby promote cell migration and settlement. Induction of the expression of an adhesion factor may be induction of the expression of a gene encoding the adhesion factor, or may be induction of the expression of the adhesion factor.

In the present invention, inflammatory cytokines are cytokines involved in immune responses and inflammatory reactions, and include interleukin 1 (IL-1), interleukin-6 (IL-6), tumor necrosis factors (TNFs), interleukin 8 (IL-8), macrophage chemotactic factors (MCFs), interferon y (IFN-γ), colony stimulating factors (CSFs), and interleukin 10 (IL-10).

In the present invention, chemokines are cytokines that control leukocyte migration and activation, and which participate in infiltration, homing and such of leukocytes in inflammation or the like. Chemokines are classified into CXC chemokines, CC chemokines, CX3C chemokines, and C chemokines. Chemokines include MCAF/MCP-1, lymphotactin, fractalkine, IL-8 and such.

In the present invention, adhesion factors represent proteins that participate in cell adhesion, and include transmembrane glycoproteins, extracellular matrix proteins, proteoglycans and such. Adhesion factors are also called cell adhesion molecules, adhesion molecules, or adhesion proteins. Adhesion factors include the immunoglobulin superfamily, cadherin superfamily, integrin superfamily, selectin family, MAM superfamily, hyaluronic acid receptor family, LRR domain family and such.

The present invention relates to methods for directing mononuclear cells into the peripheral blood of damaged tissues in a subject, or methods for increasing mononuclear cells contained in the peripheral blood of damaged tissues in a subject, both of which comprising the step of administering adrenomedullin to a subject.

In the present invention, the effect of directing mononuclear cells into peripheral blood of damaged tissues, as well as the effect of increasing mononuclear cells contained in the peripheral blood of damaged tissues in a subject may be temporary, or may be maintained for a certain period of time. Alternatively, mononuclear cells may be promoted to migrate and settle into damaged tissues and thereby directed into peripheral blood of damaged tissues, leading to an increase of mononuclear cells in the peripheral blood.

The present invention relates to methods for decreasing lung volume, static lung compliance, or average alveolar diameter, which comprise the step of administering adrenomedullin to a subject. It is known that intractable lung diseases such as pulmonary emphysema overexpand the lung volume, and increase the static lung compliance or average alveolar diameter. The effect of decreasing the lung volume, static lung compliance, or average alveolar diameter in the present invention is preferably long-lasting, but may be temporary or may be maintained for a certain period of time.

In the present invention, "static lung compliance (Cst)" is determined using a lung pressure-volume (intrathoracic pressure x lung volume) curve (P-V curve). P-V curve is measured under a no air-flow condition by blocking an air current at each lung volume. When a lung is made to expand, it generates a force (lung elastic recoil) to return to the original state. Compliance is one of the methods for estimating lung elastic recoil. Methods of measuring the lung volume, static lung compliance, or average alveolar diameter can include methods described in the Examples of the present invention, but are not limited thereto, and measurement methods known to those skilled in the art can also be used.

The present invention relates to pharmaceutical agents comprising adrenomedullin as an active ingredient that regenerate or repair damaged tissues, promote migration and settlement of mononuclear cells into damaged tissues, direct mononuclear cells into the peripheral blood of damaged tissues, increase mononuclear cells contained in the peripheral blood of damaged tissues, newly generate or regenerate peripheral vessels of damaged tissues, or decrease the lung volume, static lung compliance, or average alveolar diameter.

Pharmaceutically acceptable carriers such as preservatives and stabilizers may be added to the pharmaceutical agents of the present invention. The term "pharmaceutically acceptable" refers to materials that do not have the above-mentioned activity themselves and can be administered together with the above-mentioned pharmaceutical agents.

As a stabilizer, about 0.2% of gelatin or dextran, 0.1 to 1.0% of sodium glutamate, about 5% of lactose, about 2% of sorbitol or the like can be used, but is not limited thereto. As a preservative, about 0.01% of thimerosal, about 0.1% of β-propionolactone or the like can be used, but is not limited thereto.

When preparing injections, pH adjusters, buffers, stabilizers, preservatives and the like are added, if necessary; and subcutaneous, intramuscular, or intravenous injections are prepared by conventional methods. Injections may be stored in containers, and then lyophilized and like to be made into solid formulations or may prepared as formulations prior to use. One dose may be stored in a container, or multiple doses may be stored in a same container.

Suitable inoculation methods are determined considering the type of pharmaceutical agent, subject to be inoculated and the like. Containers may be a vial or a pre-filled syringe product. Solutions or powdered products made by lyophilization may be used, if necessary. The doses may be for a single inoculation or multiple inoculations. The dosage may vary depending on the type, weight, and age of the subjects to be inoculated, and the administration methods, but those skilled in the art can suitably select an appropriate dose.

All prior art references cited herein are incorporated herein by reference.

### [Examples]

Hereinafter, the present invention will be specifically described with reference to Examples, but it is not to be construed as being limited thereto. In the Examples, all data are represented as average ± SEM. Comparison of parameters between groups was performed by the one-tailed ANOVA test, followed by the Newman-Keuls test. The value is considered to be statistically significant if P is < 0.05.

### [Example 1] Evaluation and measurement of the dynamics of bone marrow cells by administration of adrenomedullin

First, to investigate whether AM has an effect on the dynamics of bone marrow cells, AM or physiological saline was administered randomly to model animals. As the model animals, 48 ten-week old female C57BL/6 mice were used. The transgenic mice (C57BL/6 background) which ubiquitously express green fluorescence protein (GFP) were kindly offered by Professor Masaru Okabe (Osaka University, Japan) (Okabe M, Ikawa M, Kominami K, Nakanishi T, Nishimune Y. 'Green mice' as a source of ubiquitous green cells. FEBS Lett 1997; 407: 313-319).

AM (1 µg, 2 µg, or 5 µg in 100 µl physiological saline) was randomly administrated to the C57BL/6 mice (each, n = 8) by intraperitoneal injection every day for five days. The control mice were similarly administered with 100 µl physiological saline every day for five days (n = 8).

Furthermore, AM or physiological saline was administered to other mice (each, n= 8) with a subcutaneous osmotic mini-pump (Alzet, Palo Alto, CA) at a delivery rate of 0.05 µg/kg/min for five days. Blood samples were collected on day 5, and mononuclear cells were isolated using Histopaque-1083 (Sigma) by a method known to those skilled in the art (Asahara T, Takahashi T, Masuda H, Kalka C, Chen D, Iwaguro H, Inai Y, Silver M, Isner JM. VEGF contributes to postnatal neovascularization by mobilizing bone marrow-derived endothelial progenitor cells. EMBO J 1999; 18: 3964-3972). The mononuclear cells were counted manually and analyzed with respect to the expression of Scal-FITC (Sca-1-positive cells: BD Pharmingen, San Diego, CA). The immunofluorescence-labeled cells were analyzed by a quantitative flow cytometry using the FACSCalibur Flow Cytometer (BD Biosciences, Mountain View, CA). An antibody with the same isotype (BD Pharmingen) was used as a control.

Results of the above-mentioned analysis showed that the number of the peripheral blood mononuclear cells on day 5 was increased in a dose-dependent manner by AM administration (Fig. 1A). Significant changes were detected in all cases, from the lowest dose of AM (1 µg/animal; 120% of the physiological saline group) to the highest dose of AM (5 µg/animal; 140% of the physiological saline group). A significant increase was also detected in mice continuously administered with AM using the osmotic mini pump (131% of the physiological saline group, Fig. 1B). Further, it was confirmed that the frequency of Sca-1-positive cells was also significantly high (200% of the physiological saline group, Fig. 1C).

From these results, it was shown that AM injection increased the numbers of mononuclear cells and Sca-1-positive cells in the circulating blood. It has been shown that endothelial precursor cells in the circulating blood exist in the fraction of Sca-1-positive mononuclear cells (Asahara T, Murohara T, Sullivan A, Silver M, van der Zee R, Li T, Witzenbichler B, Schatteman G, Isner JM. Isolation of putative progenitor endothelial cells for angiogenesis. Science 1997; 275: 964-967). Therefore, AM is considered to induce release of endothelial precursor cells from the bone marrow into the circulating blood.

So far, AM has been shown to induce activation of endothelial nitric oxide synthase via the phosphatidylinositol 3-kinase/Akt-dependent pathway (Nishimatsu H, Suzuki E, Nagata D, Moriyama N, Satonaka H, Walsh K, Sata M, Kangawa K, Matsuo H, Goto A, et al. Adrenomedullin induces endothelium-dependent vasorelaxation via the phosphatidylinositol 3-kinase/Akt-dependent pathway in rat aorta. Circ Res 2001; 891: 63-70). It has also been shown that recruitment of bone marrow cells is dependent on local secretion of matrix metalloprotease 9 (Heissig B, Hattori K, Dias S, Friedrich M, Ferris B, Hackett NR, Crystal RG, Besmer P, Lyden D, Moore MA, et al. Recruitment of stem and progenitor cells from the bone marrow niche requires MMP-9 mediated release of kit-ligand. Cell 2002; 109: 625-637), and in recent years that the nitric oxide activates matrix metalloprotease-9 by S-nitrosylation (Gu Z, Kaul M, Yan B, Kridel SJ, Cui J, Strongin A, Smith JW, Liddington RC, Lipton SA. S-nitrosylation of matrix metalloproteinases: signaling pathway to neuronal cell death. Science 2002; 297: 1186-1190). Furthermore, it has been shown that endothelial nitric oxide synthase is indispensable to recruitment of stem cells and precursor cells (Aicher A, Heeschen C, Mildner-Rihm C, Urbich C, Ihling C, Technau-Ihling K, Zeiher AM, Dimmeler S. Essential role of endothelial nitric oxide synthase for mobilization of stem and progenitor cells. Nat Med 2003; 9: 1370-1376).

These findings as a whole suggest that matrix metalloprotease 9 is regulated by nitric oxide synthesis induced by AM, which subsequently induces the recruitment of bone marrow cells.

### [Example 2] Evaluation of the homing and differentiation of bone marrow cells

C57BL/6GFP-positive bone marrow chimeric mice were established for evaluating the dynamics of bone marrow cells.

The above-mentioned bone marrow chimeric mice were produced by a method known to those skilled in the art (Sata M, Saiura A, Kunisato A, Tojo A, Okada S, Tokuhisa T, Hirai H, Makuuchi M, Hirata Y, Nagai R. Hematopoietic stem cells differentiate into vascular cells that participate in the pathogenesis of atherosclerosis. Nat Med 2002; 8: 403-409). To put it briefly, a lethal dose of radiation (900 cGy) was applied to wild-type recipient C57BL/6 mice, and bone marrow cells (3x10⁶ cells/300 µl) derived from the GFP mouse were transplanted through the tail vein. In order to quantify the reconstitution of mouse bone marrow, mononuclear cells and bone marrow cells in the peripheral blood were analyzed by flow cytometry four weeks after the bone marrow transplant. The percentages of the GFP-positive peripheral blood mononuclear cells and bone marrow cells in the chimeric mice were 87% to 93% and 85% to 90%, respectively, showing that the original stem cell group was replaced by the donor cell group.

Four weeks after the bone marrow transplant, swine pancreatic elastase (200 units/kg; Sigma, St. Louis, MO) was injected intratracheally to the chimeric mice. These mice were divided into two groups: the elastase-treated mice to be administered with physiological saline, and those to be administered with AM (each, n = 5). It has been fully demonstrated so far that pulmonary emphysema is induced by an intratracheal injection of protease such as elastase to experimental animals (Snider GL, Lucey EC, Stone PJ. Animal models of emphysema. Am Rev Respir Dis 1986; 133: 149-169).

The remaining 42 mice were used to examine whether AM improves lung structure and lung function in elastase-induced pulmonary emphysema. The mice were intratracheally injected with swine pancreatic elastase (200 units/kg) or physiological saline at random, and assigned to continuous infusion of either AM or physiological saline. Based on this protocol, the following three groups were prepared: the sham treated mice administrated with physiological saline (the sham treated group, n = 14), elastase-treated mice administered with physiological saline (the physiological saline group, n = 14), and elastase-treated mice administrated with AM (the AM group, n = 14). Each of the above-mentioned Examples was carried out according to the guidelines of Animal Management Ethics Committee of the National Cardiovascular Center (Osaka, Japan).

28 days after the injection of elastase (n = 5) or saline solution (n = 5), frozen sections were prepared from the lungs of the GFP-positive chimeric mice. The number of GFP-positive cells in the alveolar wall was counted and the result was indicated as the number per 100 alveoli. Alveolar epithelial cells were identified using mouse monoclonal antibodies, anti-cytokeratin 5 and 8 (Chemicon, Temecula, CA), and a goat anti-mouse antibody (Molecular Probes, Eugene, OR) conjugated to Alexa fluor 633 (Ishizawa K, Kubo H, Yamada M, Kobayashi S, Numasaki M, Ueda S, Suzuki T, Sasaki H. Bone marrow-derived cells contribute to lung regeneration after elastase-induced pulmonary emphysema. FEBS Lett 2004; 556: 249-252). Vascular endothelial cells were identified using a rabbit polyclonal antibody (DAKO, Copenhagen, Denmark) produced against von Willebrand factor (vWF) and a goat anti-rabbit antibody (Molecular Probes) conjugated to Alexa fluor 633 (Itoh T, Nagaya N, Murakami S, Fujii T, Iwase T, Ishibashi-Ueda H, Yutani C, Yamagishi M, Kimura H, Kangawa K. C-Type Natriuretic Peptide Ameliorates Monocrotaline-induced Pulmonary Hypertension in Rats. Am J Respir Crit Care Med 2004; 170: 1204-1211). The numbers of GFP/cytokeratin double-positive cells and GFP/vWF double-positive cells were counted, and the results were indicated as the number per 100 alveoli. Histological examination was conducted in a blind manner by three observers.

As a result of the above, it was shown that GFP-positive cells were detected in the alveolar wall on day 28 after the elastase injection. GFP/cytokeratin double-positive cells were also detected in the alveolar wall (Fig. 2A). A semiquantitative analysis showed that the number of GFP positive-cells incorporated in the lung in the AM group is significantly larger than that in the physiological saline group (Fig. 2B). Further, the number of GFP/cytokeratin double-positive cells in the AM group was significantly larger than that in the physiological saline group (Fig. 2C).

These results revealed that AM increases the number of bone marrow-derived cells incorporated in the elastase-administered lungs. Recent researches have shown that bone marrow precursor cells contribute to regeneration of alveoli and vasculature (Ishizawa K, Kubo H, Yamada M, Kobayashi S, Numasaki M, Ueda S, Suzuki T, Sasaki H. Bone marrow-derived cells contribute to lung regeneration after elastase-induced pulmonary emphysema. FEBS Lett 2004; 556: 249-252; Yamada M, Kubo H, Kobayashi S, Ishizawa K, Numasaki M, Ueda S, Suzuki T, Sasaki H. Bone marrow-derived progenitor cells are important for lung repair after lipopolysaccharide-induced lung injury. J Immunol 2004; 172: 1266-1272). Therefore, it is suggested that AM promotes lung regeneration by increasing the number of alveolar epithelial cells derived from the bone marrow in the elastase-administered lungs.

### [Example 3] Morphological and functional analyses of elastase-treated mice administered with AM.

It has been sufficiently demonstrated so far that pulmonary emphysema is induced by intratracheal injection of protease such as elastase to experimental animals (Snider GL, Lucey EC, Stone PJ. Animal models of emphysema. Am Rev Respir Dis 1986; 133: 149-169). In fact, in the present invention, findings about lung functions (increase in lung volume and static lung compliance) and morphological findings (increase in the average alveolar diameter) confirmed that emphysematous changes were induced in the lungs by intratracheal injection of elastase, and these findings are consistent with the results from previous studies (Hayes JA, KorthyA, Snider GL. The pathology of elastase-induced panacinar emphysema in hamsters. J Pathol 1975; 117: 1-14; Kuraki T, Ishibashi M, Takayama M, Shiraishi M, Yoshida M. A novel oral neutrophil elastase inhibitor (ONO-6818) inhibits human neutrophil elastase-induced emphysema in rats. Am J Respir Crit Care Med 2002; 166: 496-500). 28 days after the injection of elastase or physiological saline, the mice were anesthetized and their static lung compliance was measured using a computer-controlled small animal ventilator (FlexiVent; Scireq, Montreal, PQ, Canada) (n = 7). Lungs were excised and then fixed for 24 hours, while the transpulmonary pressure was maintained at 25 cm H₂O (n = 7). The lung volume was measured by Scherle's method (Scherle W. A simple method for volumetry of organs in quantitative stereology. Mikroskopie 1970; 26: 57-60). The mean linear intercept, a morphometric parameter of pulmonary emphysema, was calculated for 20 randomly selected regions with a light microscope using a method known to those skilled in the art (Thurlbeck WM. Internal surface area and other measurements in emphysema. Thorax 1967; 22: 483-496). Morphological examination was conducted in a blind manner by three observers.

Further, paraffin sections were obtained from the lungs (n = 7), and the tissue sections were stained for vWF to examine whether AM induced angiogenesis in the elastase-administered lungs. Three observers counted the number of vWF-positive blood vessels per mm² in a blind test manner.

As a result of the above, development of air space enlargement accompanied by destruction of the alveolar wall was observed in the physiological saline group on day 28 after the elastase injection (Fig. 3A). However, it was found that administration of AM decreased these histological changes in the elastase-injected mice. The average alveolar diameter in the physiological saline group was significantly larger than that in the sham treated group (Fig. 3B), but the magnitude of this difference was reduced by AM.

Moreover, GFP/vWF double-positive cells were observed in the alveolar wall (Fig. 4A), and the number of these cells in the AM group was larger than that in the physiological saline group (Fig. 4B). As a whole, the number of vWF-positive lung blood vessels decreased on day 28 after the elastase injection (Figs. 4C and 4D). However, AM injection significantly increased the number of the vWF-positive lung blood vessels in the elastase-administered lungs.

Furthermore, on day 28 after the elastase injection, the lung volume in the physiological saline group was found to be significantly larger than that in the sham treated group (Fig. 5A). The magnitude of the increase in lung volume was reduced by AM. It was also shown that the static lung compliance in the physiological saline group was significantly larger, and that this change was significantly reduced by AM (Fig. 5B).

From the above, AM was found to promote regeneration of alveoli and lung vasculature and improve lung structure and lung functions in the elastase-administered mice.

### [Example 4] Dynamic analysis of bone marrow-derived cells in a rat model of myocardial infarction administered with AM

Acute myocardial infarction was produced in GFP chimeric rats (rats expressing GFP in the bone marrow cells) by thoracotomy and then ligation of the anterior descending coronary artery (Fujii T. et al., Am J Physiol Heart Circ Physiol. 288(3): H1444-50, 2005).

After local ischemia is created in the heart by ligation, 0.05 µg/kg/min of AM (the AM group) or 0.9% physiological saline (the control group) was subcutaneously administered for seven days via an osmotic pressure pump.

After acute myocardial infarction was produced, the distributions of bone marrow-derived cells (confirmed by GFP expression), vascular endothelial cells (confirmed by vWF, an endothelial cell marker), and cardiac muscle cells (confirmed by desmin, a cardiac muscle cell marker) in the myocardial infarct area were confirmed. Further, the total sum of the GFP-expressing cell number, vWF-expressing cell number, and desmin-expressing cell number was examined to confirm the states of regeneration of blood vessel and cardiac muscle in the AM group and control group.

As a result, it was shown that after acute myocardial infarction, bone marrow-derived cells arrived and accumulated in the ischemic cardiac muscle and then blood vessels were formed (Figs. 6A and 6C). In addition, AM administration after acute myocardial infarction was found to significantly increase the revascularization by bone marrow cells (Fig. 6B). It was also shown that a portion of bone marrow-derived cells settled in the ischemic cardiac muscle, and simultaneously expressed desmin, a cardiac muscle cell marker, and GFP, a bone marrow-derived cell marker (Figs. 7A and 7B). Furthermore, AM administration after acute myocardial infarction was found to significantly enhance the regeneration of cardiac muscles by bone marrow-derived cells (Fig. 7C).

### Industrial Applicability

Capillary network is inevitably destroyed in the process of pulmonary emphysema because capillaries account for a considerable proportion of the alveolar wall volume. Thus, it is important to regenerate not only alveoli but also the lung vasculature simultaneously for repairing emphysematous pathology. In the present invention, the number of GFP/vWF double-positive cells was increased by AM injection, and this result suggested that AM increased vascular endothelial cells derived from the bone marrow. Furthermore, recent studies have shown that AM exerts angiogenic effects by activating Akt, mitogen-activated protein kinase/extracellular signal-regulated kinase 1/2, and focal adhesion kinase in endothelial cells. In the present invention, AM significantly increased the number of the lung blood vessels in the elastase-treated mice. Therefore, the AM-induced angiogenesis in the lung is thought to contribute to lung regeneration in the elastase-treated mice.

When adrenomedullin was continuously infused into the damaged tissue in the lung by a method of the present invention, the elastase-induced pulmonary emphysema was reduced. In addition, when adrenomedullin was infused into the damaged tissue in myocardial infarction, it was found to promote the arrival and accumulation of bone marrow-derived cells in the ischemic cardiac muscle, and increase angiogenesis and cardiac muscle neogenesis. These useful effects seem to be mediated by recruitment of bone marrow-derived cells and regeneration of alveoli, cardiac muscle cells, and vasculature.

The present invention is the first trial in the world that applies adrenomedullin to regeneration of pulmonary emphysema, i.e., regeneration of alveoli and lung blood vessels, and these methods can be excellent methods for regenerating or treating diseases accompanied by fatal damages to peripheral vessels, such as old myocardial infarction and pulmonary emphysema. Enhancement of natural healing ability, development of new treatment methods, and rebuilding of defective functions for these diseases are expected to not only improve the quality of life in patients, but also reduce health care costs by inpatient rehabilitation.

## Claims

1. Use of adrenomedullin for the preparation of a medicament for regenerating or repairing a damaged tissue in an alveolus or cardiac muscle in a subject.

2. The use of claim 1, wherein the damaged tissue in the subject is a damaged tissue in an intractable cardiopulmonary disease.

3. The use of claim 1, wherein the damaged tissue in the subject is a damaged tissue in pulmonary emphysema.

4. The use of claim 1, wherein the damaged tissue in the subject is a damaged tissue in any one of myocardial infarction, myocarditis, and myocardosis.

5. The use of claim 3, wherein adrenomedullin decreases lung volume, static lung compliance, or average alveolar diameter in a subject.

6. A pharmaceutical agent comprising adrenomedullin for use in regenerating or repairing a damaged tissue in an alveolus or cardiac muscle in a subject.

7. The pharmaceutical agent for use in regenerating or repairing a damaged tissue in an alveolus or cardiac muscle in a subject according to claim 6, wherein the damaged tissue is in an intractable cardiopulmonary disease.

8. The pharmaceutical agent for use in regenerating or repairing a damaged tissue in an alveolus or cardiac muscle in a subject according to claim 6, wherein the damaged tissue is in pulmonary emphysema.

9. The pharmaceutical agent for use in regenerating or repairing a damaged tissue in an alveolus or cardiac muscle in a subject according to claim 6, wherein the damaged tissue is in any one of myocardial infarction, myocarditis, and myocardosis.

10. The pharmaceutical agent for use in regenerating or repairing a damaged tissue in an alveolus or cardiac muscle in a subject according to claim 8, wherein adrenomedullin decreases lung volume, static lung compliance, or average alveolar diameter.

## Patentansprüche

1. Verwendung von Adrenomedullin für die Herstellung eines Medikaments zum Regenerieren oder Reparieren geschädigten Gewebes in einer Alveole oder einem Herzmuskel eines Patienten.

2. Verwendung nach Anspruch 1, wobei das geschädigte Gewebe des Patienten geschädigtes Gewebe bei einer hartnäckigen Herzlungenkrankheit ist.

3. Verwendung nach Anspruch 1, wobei das geschädigte Gewebe des Patienten geschädigtes Gewebe bei einem Lungenemphysem ist.

4. Verwendung nach Anspruch 1, wobei das geschädigte Gewebe des Patienten geschädigtes Gewebe bei einem von Herzinfarkt, Herzmuskelentzündung und Myokardose ist.

5. Verwendung nach Anspruch 3, wobei Adrenomedullin das Lungenvolumen, die statische Lungencompliance oder den mittleren Alveolardurchmesser eines Patienten verringert.

6. Pharmazeutisches Mittel, das Adrenomedullin aufweist, zur Verwendung beim Regenerieren oder Reparieren von geschädigtem Gewebe in einer Alveole oder einem Herzmuskel eines Patienten.

7. Pharmazeutisches Mittel zur Verwendung beim Regenerieren oder Reparieren von geschädigtem Gewebe in einer Alveole oder einem Herzmuskel eines Patienten nach Anspruch 6, wobei das geschädigte Gewebe bei einer hartnäckigen Herzlungenkrankheit vorliegt.

8. Pharmazeutisches Mittel zur Verwendung beim Regenerieren oder Reparieren von geschädigtem Gewebe in einer Alveole oder einem Herzmuskel eines Patienten nach Anspruch 6, wobei das geschädigte Gewebe bei einem Lungenemphysem vorliegt.

9. Pharmazeutisches Mittel zur Verwendung beim Regenerieren oder Reparieren von geschädigtem Gewebe in einer Alveole oder einem Herzmuskel eines Patienten nach Anspruch 6, wobei das geschädigte Gewebe bei einem von Herzinfarkt, Herzmuskelentzündung und Myokardose vorliegt.

10. Pharmazeutisches Mittel zur Verwendung beim Regenerieren oder Reparieren von geschädigtem Gewebe in einer Alveole oder einem Herzmuskel eines Patienten nach Anspruch 8, wobei Adrenomedullin das Lungenvolumen, die statische Lungencompliance oder den mittleren Alveolardurchmesser verringert.

## Revendications

1. Utilisation d'adrénomédulline pour la préparation d'un médicament pour régénérer ou réparer un tissu endommagé dans une alvéole ou le muscle cardiaque chez un sujet.

2. Utilisation selon la revendication 1, dans laquelle le tissu endommagé chez le sujet est un tissu endommagé dans une maladie cardio-pulmonaire réfractai re.

3. Utilisation selon la revendication 1, dans laquelle le tissu endommagé chez le sujet est un tissu endommagé dans un emphysème pulmonaire.

4. Utilisation selon la revendication 1, dans laquelle le tissu endommagé chez le sujet est un tissu endommagé dans l'un quelconque parmi un infarctus du myocarde, une myocardite et une myocardie.

5. Utilisation selon la revendication 3, dans laquelle l'adrénomédulline diminue le volume des poumons, la compliance pulmonaire statique ou le diamètre alvéolaire moyen chez un sujet.

6. Agent pharmaceutique comprenant de l'adrénomédulline pour une utilisation dans la régénération ou la réparation d'un tissu endommagé dans une alvéole ou le muscle cardiaque chez un sujet.

7. Agent pharmaceutique pour une utilisation dans la régénération ou la réparation d'un tissu endommagé dans une alvéole ou le muscle cardiaque chez un sujet selon la revendication 6, dans lequel le tissu endommagé est dans une maladie cardio-pulmonaire réfractaire.

8. Agent pharmaceutique pour une utilisation dans la régénération ou la réparation d'un tissu endommagé dans une alvéole ou le muscle cardiaque chez un sujet selon la revendication 6, dans lequel le tissu endommagé est dans un emphysème pulmonaire.

9. Agent pharmaceutique pour une utilisation dans la régénération ou la réparation d'un tissu endommagé dans une alvéole ou le muscle cardiaque chez un sujet selon la revendication 6, dans lequel le tissu endommagé est dans l'un quelconque parmi un infarctus du myocarde, une myocardite et une myocardie.

10. Agent pharmaceutique pour une utilisation dans la régénération ou la réparation d'un tissu endommagé dans une alvéole ou le muscle cardiaque chez un sujet selon la revendication 8, dans lequel l'adrénomédulline diminue le volume des poumons, la compliance pulmonaire statique ou le diamètre alvéolaire moyen.
